(19) **Europäisches Patentamt**
**European Patent Office**
**Office européen des brevets**

(11) **EP 2 115 011 B1**

(12) **EUROPEAN PATENT SPECIFICATION**

(45) Date of publication and mention
of the grant of the patent:
**27.07.2011 Bulletin 2011/30**

(21) Application number: **07858089.1**

(22) Date of filing: **21.12.2007**

(51) Int Cl.:
***C08B 37/00*** *(2006.01)*        ***C08L 5/08*** *(2006.01)*
***A61K 47/38*** *(2006.01)*

(86) International application number:
**PCT/EP2007/064478**

(87) International publication number:
**WO 2008/077949 (03.07.2008 Gazette 2008/27)**

(54) **CYTOCOMPATIBLE STABLE GEL COMPOSITIONS AS SUPPORTS FOR CELL GROWTH**

ZYTOKOMPATIBLE STABILE GELZUSAMMENSETZUNGEN ALS TRÄGER FÜR ZELLWACHSTUM

COMPOSITIONS DE GEL STABLES ET CYTOCOMPATIBLES EN TANT QUE SUPPORTS POUR LA CROISSANCE CELLULAIRE

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **27.12.2006  IT MI20062513**

(43) Date of publication of application:
**11.11.2009  Bulletin 2009/46**

(73) Proprietor: **UNIVERSITA' DEGLI STUDI DI PARMA 43100 Parma (IT)**

(72) Inventors:
• **ROMANI, Antonello**
**I-43100 Parma (IT)**
• **BORGHETTI, Angelo**
**I-43100 Parma (IT)**
• **BETTINI, Ruggero**
**I-43056 San Polo Di Torrile (IT)**

(74) Representative: **Gervasi, Gemma et al**
**Notarbartolo & Gervasi S.p.A.**
**Corso di Porta Vittoria 9**
**20122 Milano (IT)**

(56) References cited:
**US-A1- 2004 047 892     US-B1- 6 344 488**

## EP 2 115 011 B1

### Description

### Field of the invention

The present invention relates to stable chitosan gel compositions for constructing three-dimensional biodegradable films and/or matrices of high cyto- and biocompatibility, able to act as substrates for both *in vivo* and *in vitro* growth of various histotypes (mesenchymal, endothelial and epithelial).

More specifically the present invention relates to chitosan compositions comprising D-(+) saccharose and/or D-(+) raffinose, alkaline and/or alkaline-earth metal phosphates and chlorides, usable for obtaining gel supports suitable for cell growth (adhesion, survival, proliferation and maintenance of differentiation). These compositions are useful for constructing artificial substrates for cell growth, which can also be used in autologous and heterologous implantology.

### State of the art

Organ or tissue failures account for about one half the health costs incurred by most western countries. Traumas and illnesses of various kinds can involve tissue such as the skin, bone or bone marrow, or affect complex organs such as kidneys, the liver or heart. The therapeutic options available to physicians for organic diseases are medical therapy, reparative or reconstructive surgery, prosthetic or mechanical supports, and finally transplants. Medical therapy is often complicated by serious side-effects, whereas prosthetic or mechanical supports in most cases do not lead to complete recovery of the functions of the damaged organ or tissue, neither can they halt the progression of the illness, and are often a vehicle for systemic infections. With regard to transplants, most of the problems are due to donor shortages and the chronic immunosuppressive therapy to which patients are subjected. In the light of these problems, new solutions must be found that can bring about a satisfactory recovery and maintenance of the specific organ or tissue functions.

One of the most interesting ways is the emerging field of tissue engineering (H.Shin et al. Biomaterials 24 (2003) 4353-4364) which has been defined as *"...an interdisciplinary field of research that applies the principles of engineering and biological sciences to the development of biological substitutes that restore, maintain or improve tissue or organ function".* At the basis of this new discipline, there is profound knowledge of embryology and physiology directed particularly to the formation, regeneration and growth of functional and functioning tissue. This new technology involves the use of both synthetic and natural components to create or replace damaged organ or tissue parts (Nerem, Ann. Biomed. Eng. 19:529-45; 1991). This approach can be achieved by three successive steps:

(i) development of a biocompatible, biodegradable and sterilizable substrate, provided with an optimal surface on which the new tissue can grow and develop,
(ii) creation of models for studying three-dimensional tissue organisation,
(iii) supply of cellular parts or supports for implantation into the human body.

In parallel, tissue engineering can also provide suitable carriers for transporting drugs or engineered cells to the damaged organs and/or tissue.

In this respect considerable attention has been given to chitosan. Chitosan is a high molecular weight deacetylated derivative of chitin and is the second most abundant natural polymer. It is the common component of marine crustacean shells and the cell walls of certain fungi. From the structural viewpoint it is a linear polysaccharide composed of N-acetyl-glucosamine and glucosamine bound by a β-glycosidic (1-4) bond. The ratio of glucosamine to N-acetyl-glucosamine units is defined as degree of acetylation. The molecular weight can vary from 300 to more than 2,000 kD, while the degree of acetylation can vary from 30% to 98%. The cationic nature of chitosan is responsible for the electrostatic interactions with negatively charged molecules such as anionic glycosaminoglycans and proteoglycans. Various authors have described a correlation between the degree of chitosan acetylation and cellular adhesion (J Biomater Appl. 20 (2005) 157-77, Biomaterials. 25 (2004) 3973-81). Numerous researchers have studied the reaction of the body to chitosan based implants (Rucker M et al., Biomaterials. 2006, Osteoarthritis Cartilage. 13 (2005) 798-807). Generally, minimum foreign body reaction has been found with low or no fibrotic encapsulation (J Biomed Mater Res. 59 (2002) 585-90). The formation of granulation tissue associated with rapid angiogenesis has also been described (Biomaterials. 21 (2000) 2589-98). This modest inflammatory/immune response allows rapid integration of the material implanted in the host.

One of the most important characteristics of chitosan is its capacity to be modelled into various shapes and sizes. In this respect, we note the formation of films of various thickness, three-dimensional blocks, tubes and spheres (Biomaterials. 25 (2004) 779-85, Tissue Eng. 11 (2005) 1105-14, J Biomed Mater Res A. 77 (2006) 277-84). A review of the scientific literature in this respect is reported for example by A. Di Martino et al. Biomaterials 26 (2005) 5983-5990.

Chitosan is particularly indicated in the construction of three-dimensional supports of high porosity. There are various procedures for producing this type of substrate, including freeze-drying. With this procedure a porous material can be obtained with a pore size and orientation which depends on the freezing rate, the size of the ice crystals and the thermal gradient curve (Madihally et al. Biomaterials 20 (1999) 1133-1142).

Chitosan has been used in association with various substances such as alginate, hydroxyapatite, hyaluronic acid, calcium phosphate, polymethylmethacrylate (PMMA), poly(DL-lactide-co-glycolide) (PLGA), polylactic acid (PLA) and various growth factors. In general, chitosan offers a number of possibilities in the tissue engineering field.

JP 62083875, US 6719987 and WO 05089416 claim the preparation of chitosan film with anti-microbial properties.

JP 03151976 and CN 1628865 report the obtaining of composite films of chitosan and polyvinylalcohol or sodium arginate respectively.

EP 1246650 reports the construction of a flexible biodegradable chitosan film for use as a covering for wounds. This film is composed of 80% chitosan with a degree of acetylation between 70% and 88%. Likewise, JP 8269383 reports the construction of a chitosan film characterized by high mechanical resistance and good solvent resistance. This invention comprises the formation of covalent bonds between the chitosan gel and a polyamide resin functionalized with a residue able to form covalent bonds with -NH$_2$ groups of chitosan. Another Japanese patent application (JP 2004248949) in which the chitosan solution, modelled in the form of a film by drying, comprises one or more components chosen from glucomannan, pullulan, carrageenan or chitosan, and polyalcohols, polyols, monosaccharides, disaccharides and oligosaccharides.

JP 11092504 claims compounds with bactericide action consisting of complex chitosan and sugar chains obtained by copolymerisation. The sugar in question is L-fucose which constitutes a ligand for specific binding sites on the surface of the bacterial cell.

EP 1232203 describes the preparation of a biocompatible three-dimensional matrix able to act as a support for cell cultures and for use as an implant. This matrix is produced by lyophilization of a chitosan solution in acidified water.

US 2003/0124172 claims a method for producing chitosan film with an increased capacity for cell adhesion. The preparation of this film, which can also take place in the presence of a biodegradable polymer such as polyglycolic acid, alginate, carrageenan and collagen, requires the addition of stabilizing agents such as aqueous alkaline solutions and buffer solutions at pH not less than 5. The addition of biologically active substances is also provided.

US 7022523 proposes a carrier for cell cultures; this invention provides a medium for cell growth consisting of an aqueous polymer gel containing chitosan covered by a layer of gelatin and/or alginic acid.

Many applications in the biomedical field and for the controlled release of substances involving the use of chitosan require the formation of a stable gel, i.e. of a three-dimensional polymer structure able to retain a determined percentage of solvent. Said stable gel can be obtained by different processes:

i) chemical crosslinking, involving the formation of covalent bonds between adjacent polymer chains for example by the effect of a reaction with glutaraldehyde;
ii) physical gelling:

- formation of hydrogen bonds between chitosan chains following neutralization of the acid polymer solution with a base;
- formation of electrostatic interactions by complexing with bivalent anions such as phosphate or borate, or with anionic surfactants.

WO 2005097871 claims compositions and a method for preparing transparent chitosan gels free of phosphate. Likewise, WO 2005079749 claims compositions based on chitosan and phosphoric esters of polyols or sugars suitable for the controlled release of therapeutic agents across mucous membranes.

US 6344488 describes the formation, controlled by temperature and dependent on pH, of ionic polysaccharide gels such as chitosan/organophosphate/water systems. Said gels also comprise the presence in their compositions of phosphoric ester salts with polyols or sugars. These compositions are intended for the encapsulation of cells or cellular materials, of drugs for forming drug delivery systems or for the production of artificial matrices containing cells to be used in the field of tissue engineering. In all the aforesaid patents, the three-dimensional structuring of the gels takes place via the formation of electrostatic interactions following complexing with the organophosphoric ester.

## Summary of the invention

The present invention provides a composition suitable for the formation a stable gel in the form of film or a three-dimensional matrix obtained by mixing specific salts and sugars with a weakly acid solution of chitosan, then neutralizing the resultant solution.

The main object of the present invention is to provide a new chitosan based composition comprising a combination of specific salts and sugars for preparing stable gels in the form of a film or three-dimensional matrix provided with an excellent cytocompatibility, biocompatibility and biodegradability, and thus suitable for acting as a support for *in*

*vitro* and *in vivo* adhesion and proliferation of cells. A further object is to provide a biocompatible and biodegradable material suitable for constructing complex three-dimensional structures for tissue and organ engineering applications, such as vascular prosthesis, bile duct prosthesis, materials for filling bone defects, aids for wound repair, reconstruction of parenchymatous organs or hemopoietic tissues. The main characteristic of this composition is its capacity to ensure optimal cell proliferation, comparable to that obtainable on plastic supports typically used for cell cultures. The advantages in terms of economics and flexibility of use are fully evident.

Said capacity is determined by the distinctive structure which the films or matrices assume due to the presence of the aforesaid components, in terms of surface characteristics, spatial organization of polymer chains, wettability, water content and surface charge. In particular it has been surprisingly found that the addition of a sugar chosen from D-(+) raffinose and/or D-(+) saccharose, a phosphate salt and one or more alkaline or alkaline-earth metal chlorides to a weakly acid solution of chitosan enables a gel to be obtained which after neutralization gives rise to films or three-dimensional matrices with excellent cytocompatibility characteristics; in contrast, the absence of said sugars or their replacement with other sugars does not enable materials with suitable structural and cytocompatibility characteristics to be obtained.

## Description of the figures

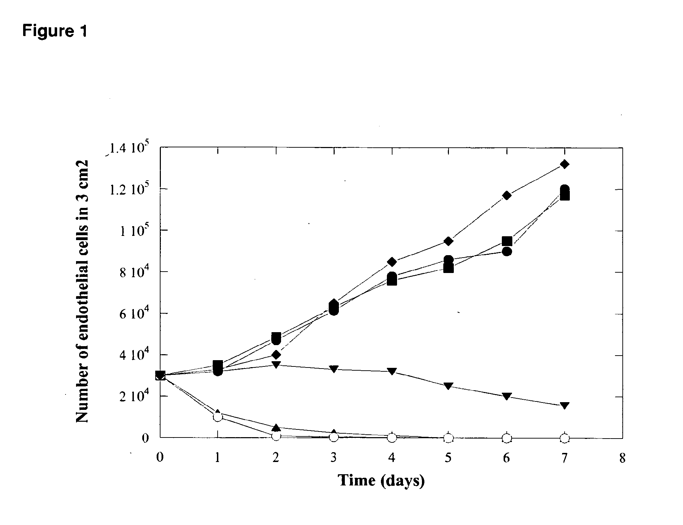

**Figure 1**: Number of endothelial cells on 3 cm$^2$ as a function of time on films of chitosan alone (inverted triangle), chitosan and salts (empty circle), chitosan containing D-(+) trehalose and salts (solid triangle), chitosan containing D-(+) raffinose and salts (solid circle) or chitosan containing D-(+) saccharose and salts (solid square), compared with cell growth on plastic dishes (Petri dishes, Corning) commonly used for cell cultures (solid diamond).

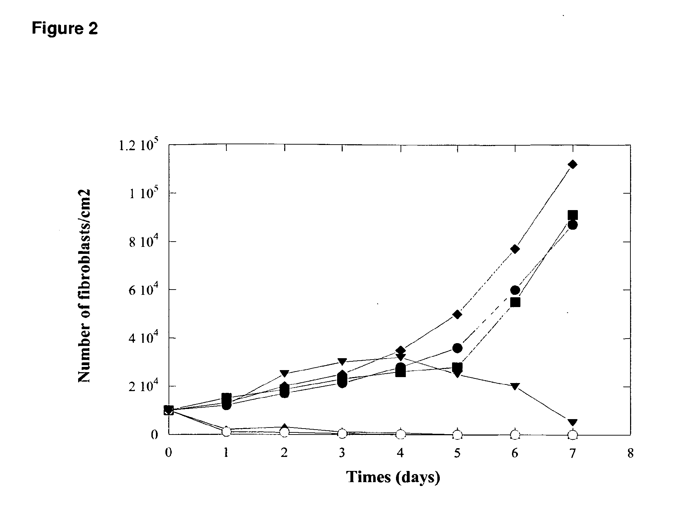

**Figure 2:** Number of fibroblasts per cm$^2$ as a function of time on films of chitosan alone (inverted triangle), chitosan and salts (empty circle), chitosan containing D-(+) trehalose and salts (solid triangle), chitosan containing D-(+) raffinose and salts (solid circle) or chitosan containing D-(+) saccharose and salts (solid square), compared with cell growth on plastic dishes (Petri dishes, Corning) commonly used for cell cultures (solid diamond).

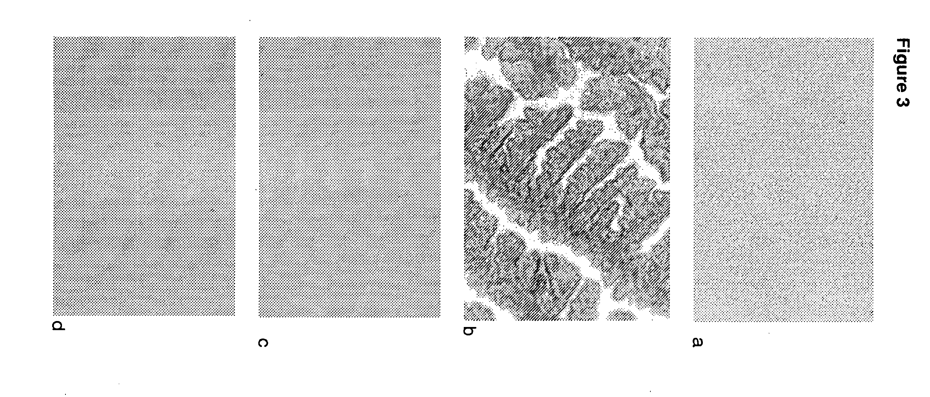

**Figure 3:** Optical microscope images (100x) of gels of: chitosan (a); chitosan and salts (b); chitosan, saccharose and salts (c); chitosan, raffinose and salts (d).

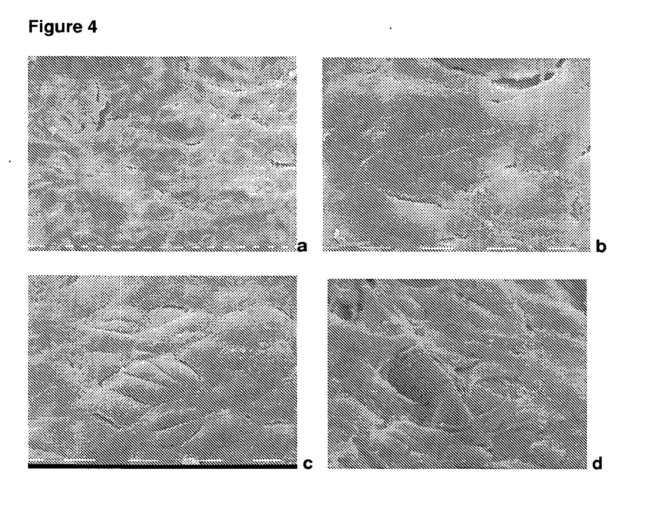

**Figure 4:** Images obtained with a scanning electron microscope after 3 weeks of co-culturing endothelial cells and fibroblasts on gels in the form of a three-dimensional matrix prepared as described in example 6. Enlargement 1500x (a-c) 2000x (d).

## Detailed description of the invention

The present invention describes compositions useful for preparing gels of high stability and high cytocompatibility, for use as a support material for the proliferation of cells which maintain their differentiation state. Said gels are produced from a solution of chitosan in water acidified with an inorganic or organic acid, preferably hydrochloric acid or acetic acid in a concentration between 0.1 and 1.1 M, and more preferably acetic acid in a concentration of 0.17 M.

The term chitosan means derivatives of chitin, or poly-N-acetyl-D-glucosamine, including polyglucosamines and glucosamine oligomers of different molecular weights in which the N-acetyl group content has been reduced by hydrolysis (deacetylation). According to the present invention the degree of acetylation which represents the percentage of N-acetyl groups removed by deacetylation, is between 40 and 98% and preferably between 60 and 95%, and even more preferably between 80 and 92%. The molecular weight of said chitosan is generally between 2 and 2,000 kDa, preferably between 25 and 1,000 kDa, and even more preferably between 100 and 900 kDa.

Unexpectedly, it has been found that the addition of D-(+) raffinose or D-(+) saccharose, phosphates (preferably mono and dibasic such as $Na_2HPO_4$, $NaH_2PO_4$, $KH_2PO_4$) and alkaline and alkaline-earth metal chlorides (preferably NaCl, KCl, $CaCl_2$, $MgCl_2$) to said aqueous acid solution of chitosan, followed by neutralization has enabled gels to be formed possessing high stability and cytocompatibility, at levels significantly above that obtainable with gels of chitosan alone or gels obtained in the absence of said combination of components.

Said gels can be further treated by methods known in the art, to obtain specific supports, preferably films or three-dimensional matrices; at the equilibrium hydration state, the films present a thickness typically between 0.2 and 0.9 mm; under the same conditions the matrices present a thickness typically between 1 and 3 mm. The film or the three-dimensional matrix obtained in accordance with the present invention is further characterized as follows:

i) lack of a microscopically visible supramolecular organization (as observable by optical microscopy);
ii) smooth surface with surface roughness not exceeding 50 nm. The term surface roughness means the average depth of depressions in the material surface as measurable by an atomic force microscope;

iii) high wettability after drying, with water contact angle less than 40°;

iv) swelling index in water exceeding 1000%. The term swelling index means the percentage ratio of weight of material at equilibrium in water minus weight of the dry material to the weight of the dry material itself.

The gel of the present invention is prepared by dissolving chitosan in a solution acidified, for example, with acetic acid having concentration between 0.1 and 0.2 M and preferably 0.17 M concentration; the chitosan is preferably used in purified form; purification can be undertaken for example as described by Struszczyk, Doctoral Thesis, University of Potsdam, Germany, November 2000; the quantity of chitosan used is such as to obtain a concentration preferably of between 0.1 and 4% w/v, more preferably between 1 and 3 % w/v and still more preferably 2% w/v, where w means the weight of chitosan in the dry state and v means the volume of the acid solution measured after adding the chitosan.

After mixing the solution by a suitable mixer until a clear and viscous solution is obtained, D-(+) raffinose and/or D-(+) saccharose, one or more phosphate salts and one or more alkaline and/or alkaline-earth metal chlorides are added. Preferably, these additions are made under the following conditions:

- the sugar is added in a concentration of between 0.003 and 0.5 M, more preferably between 0.03 and 0.4 M, and still more preferably between 0.2 and 0.3 M. If a combination of D-(+) raffinose and D-(+) saccharose is used, said molarity ranges refer to the sum of molarities of the respective sugars.
- the phosphate salt is chosen from one or more of the following salts:

  dibasic sodium phosphate ($Na_2HPO_4$) in a quantity such as to obtain a concentration of between 0.1 and 35 mM, and more preferably between 5 and 10 mM;
  monobasic sodium phosphate ($NaH_2PO_4$) in a quantity such as to obtain a concentration of between 1 and 125 mM, and more preferably between 15 and 30 mM;
  monobasic potassium phosphate ($KH_2PO_4$) in a quantity such as to obtain a concentration of between 0.1 and 5 mM, and more preferably between 0.5 and 2.5 mM. In a particularly preferred variant, the composition contains monobasic and
  dibasic phosphate salts simultaneously.

- The alkaline or alkaline-earth metal chlorides are chosen from one or more of the following salts:

  sodium chloride (NaCl) in a quantity such as to obtain a concentration of between 1 and 300 mM, and more preferably between 50 and 200 mM;
  potassium chloride (KCl) in a quantity such as to obtain a concentration of between 0.1 and 5 mM, and more preferably between 1 and 3.5 mM;
  calcium chloride ($CaCl_2$) in a quantity such as to obtain a concentration of between 0.1 and 2 mM, and more preferably between 0.5 and 1.5 mM; magnesium chloride ($MgCl_2$) in a quantity such as to obtain a concentration between 0.1 and 2 mM, and more preferably between 0.5 and 1.5 mM. In a particularly preferred variant, the composition contains all the aforesaid chlorides. All said sugar and salt molarities are calculated on the initial chitosan acid solution.

A characteristic aspect of the present invention is that the gel forms by the action of said phosphate and sugar molecules as mutually independent units, i.e. not bound together by covalent bonds, such as ester bonds. The interaction observed in the present invention has also been found to be specific for the two aforesaid sugars. In this respect, as indicated in the experimental part, their replacement by other sugars provides compositions with insufficient cytocompatibility characteristics.

To obtain the gel in the form of film or a three-dimensional matrix of the present invention, it is essential to firstly prepare the acidified chitosan solution and then add the salts and the sugar; the specific sequence of adding sugars and salts to the acidified chitosan is not however crucial; they can be therefore added in any order.

The composition, following addition of the sugars and salts, is then treated with a base to eliminate the acid excess and bring the system to neutrality; the composition is firstly dried before being treated with the base; the base used is not crucial and can be chosen for example from aqueous KOH or NaOH solutions. Conveniently, before undertaking the neutralization, the composition is placed in suitable moulds determining the final form of the gelled support. For example, to obtain films or three-dimensional matrices, the composition obtained as heretofore described, can be inserted into glass moulds of geometry suitable for obtaining thin layers with layers of predetermined thickness in accordance with the following modalities.

- Film preparation

The mould containing the solution as a thin layer is placed in an air circulation oven at a temperature between 43 and 50°C and preferably at 45°C until it is dry.

The dried film is placed in an aqueous solution of potassium hydroxide (KOH) having concentration between 100 and 900 mM and preferably between 400 and 600 mM for at least 6 hours. The film is then washed with portions of distilled water until wash waters of neutral pH are obtained.

The film obtained in this manner is preserved in distilled water until used.

- Three-dimensional matrix preparation

The mould containing the solution in a layer of suitable thickness is treated by freeze gelation as described by M.H. Ho et al. (Biomaterials 25 (2004) 129-138), by freezing to a temperature between -20 and -190°C to obtain matrices of suitable porosity (S. V. Madihally & H. W. T. Matthew Biomaterials 20 (1999) 1133-1142).

Freezing is followed by a step of neutralizing the excess acetic acid which has remained trapped in the frozen three-dimensional structure, with a solution of alcoholic potash (40% v/v of an aqueous solution of KOH (60% w/v) in 96% ethanol v/v). This neutralization is conducted at -20°C and leads to progressive formation of the gel. The alcoholic potash solution is then progressively replaced by small portions of distilled water (250 microlitres/minute), maintaining the system under agitation until complete replacement of the alcoholic potash solution by distilled water.

The matrix obtained in this manner is preserved in distilled water until used.

If the use of the gel in film or matrix form involves contact with living material, said film or matrix is further sterilized by placing it in a container containing a buffer solution at neutral or physiological pH. This container is then sterilized in an autoclave in accordance with the procedure described in the Official Pharmacopeia of the Italian Republic (OP XI ed.) chapter 5.1 page 230. Said sterilization process does not produce any modifications in the gel characteristics. Some examples relative to the preparation are described hereinafter by way of non-limiting example, to illustrate the characteristics of the invention.

Example 1 (reference)

*Preparation of a gel in film form of chitosan alone*

*a) Purification of chitosan*

2 g of chitosan (ACEF, Fiorenzuola d'Arda (PC), minimum degree of acetylation 90%) was dissolved in 100 ml of a saline solution containing 0.9% of NaCl. The solution was placed on a magnetic agitator for 20 minutes. 1 ml of glacial acetic acid was then added. After leaving this solution under agitation for 1 hour at ambient temperature, it was placed in a sterile hood under vacuum to allow the trapped air bubbles to escape. A 500 mM KOH solution was prepared for use in the precipitation step. The potash solution volume was about one half the volume of the chitosan solution. Using a peristaltic pump set at a rate of 1 ml/minute, the alkaline solution was dropped into the chitosan solution under agitation. After complete precipitation of the chitosan, an ivory-white mass formed and was collected, washed with distilled water (until neutral pH wash waters were obtained) and finally dehydrated in absolute alcohol. The material collected after dehydration was then placed in an oven at a temperature not exceeding 45°C until completely dried. The dried chitosan (equal to 85-90% of the starting weight of the chitosan) was ground in a knife mill and sieved to obtain a powder with a mean diameter of 200-300 $\mu$M.

*b) Preparation of the purified chitosan solution*

A suitable quantity of purified chitosan was dissolved in water acidified with acetic acid (acid concentration = 0.17 M) and placed under agitation until the chitosan powder was completely dissolved. The solution was then filtered through two filters of 1.2 and 0.8 $\mu$m to remove all foreign particles and to obtain a clear solution of straw yellow colour.

*c) Preparation of films of chitosan alone*

Using a silicone pen (Liquid Blocker, Daido Sangyio Co. Ltd, Japan) rectangles of size 3 x 1 cm were drawn on microscope slides. One ml of solution obtained by the procedure under point b) was distributed homogeneously within the previously drawn rectangles. The slides containing the solution were fully dried in an oven at 45°C for 24 hours. The dried film was placed in an aqueous solution of potassium hydroxide (KOH) of 500 mM concentration for 6 hours. The film was then washed with portions of distilled water until the wash waters were of neutral pH.

*d) Sterilization of the films*

The film was sterilized by placing it into a container containing a buffer solution at neutral pH. This container was then sterilized in an autoclave at 121°C for 15 minutes.

Example 2

*Preparation of a gel in film form starting from chitosan, D-(+) raffinose and salts*

*a) Chitosan purification*

Chitosan purification was carried out by the procedure described under point (a) of example 1.

*b) Preparation of the purified chitosan solution*

The preparation followed the procedure described under point (b) in example 1. D-(+) raffinose pentahydrate (MP Biomedicals, Ohio, USA) was then added in a quantity such as to obtain a 0.2 M concentration and the solution was agitated at 37°C until the sugar crystals had completely dissolved. Dibasic sodium phosphate ($Na_2HPO_4$) was then added in a quantity such as to obtain a 7.5 mM concentration, monobasic sodium phosphate ($NaH_2PO_4$) in a quantity such as to obtain a 22 mM concentration, monobasic potassium phosphate ($KH_2PO_4$) in a quantity such as to obtain a 1.5 mM concentration, sodium chloride (NaCl) in a quantity such as to obtain a 125 mM concentration and potassium chloride (KCl) in a quantity such as to obtain a 2 mM concentration. The solution was agitated at 37°C for 1 hour and filtered through 1.2 and 0.8 $\mu$m filters.

*c) Chitosan film preparation*

The film was prepared using the method described under point c) of example 1.

*d) Film sterilization*

The film was sterilized by placing it in a container containing a buffer solution at neutral pH. This container was then sterilized in an autoclave at 121°C for 15 minutes.

Example 3

*Preparation of a gel in film form starting from chitosan, D-(+) saccharose and salts*

*a) Chitosan purification*

Chitosan purification was carried out by the procedure described under point (a) of example 1.

*b) Preparation of the purified chitosan solution*

The preparation of the solution followed the procedure described under point (b) in example 1. D-(+) saccharose (ICN Biomedicals Inc., Ohio, USA) was then added in a quantity such as to obtain a 0.29 M concentration and the solution was agitated at 37°C until the sugar crystals had completely dissolved. Dibasic sodium phosphate ($Na_2HPO_4$) was then added in a quantity such as to obtain a 8 mM concentration, monobasic sodium phosphate ($NaH_2PO_4$) in a quantity such as to obtain a 25 mM concentration, monobasic potassium phosphate ($KH_2PO_4$) in a quantity such as to obtain a 2 mM concentration, sodium chloride (NaCl) in a quantity such as to obtain a 100 mM concentration and potassium chloride (KCl) in a quantity such as to obtain a 1.8 mM concentration. Calcium chloride ($CaCl_2$) was then added in a quantity such as to obtain a 1 mM concentration and magnesium chloride ($MgCl_2$) in a quantity such as to obtain a 1.1 mM concentration. The solution was agitated at 37°C for 1 hour and filtered through 1.2 and 0.8 $\mu$m filters.

*c) Chitosan film preparation*

The film was prepared using the method described under point (c) of example 1.

*d) Film sterilization*

The film was sterilized by placing it in a container containing a buffer solution at neutral or physiological pH. This container was then sterilized in an autoclave at 121 °C for 15 minutes.

Example 4 (reference)

*Preparation of a gel in film form starting from chitosan and salts*

*a) Chitosan purification*

Chitosan purification was carried out by the procedure described under point (a) of

example 1.

*b) Preparation of the purified chitosan solution*

The preparation followed the procedure described under point (b) in example 1. Dibasic sodium phosphate ($Na_2HPO_4$) was then added in a quantity such as to obtain a 7.5 mM concentration, monobasic sodium phosphate ($NaH_2PO_4$) in a quantity such as to obtain a 22 mM concentration, monobasic potassium phosphate ($KH_2PO_4$) in a quantity such as to obtain a 1.5 mM concentration, sodium chloride (NaCl) in a quantity such as to obtain a 125 mM concentration and potassium chloride (KCl) in a quantity such as to obtain a 2 mM concentration. The solution was agitated at 37°C for 1 hour and filtered through 1.2 and 0.8 $\mu$m filters.

*c) Chitosan film preparation*

The film was prepared using the method described under point (c) of example 1.

*d) Film sterilization*

The film was sterilized by placing it in a container containing a buffer solution at neutral pH. This container was then sterilized in an autoclave at 121 °C for 15 minutes.

Example 5

*Preparation of a gel in film form starting from chitosan, a monosaccharide and salts*

The procedure for preparing the films containing a monosaccharide (glucose or mannose) in place of the D-(+) raffinose or the D-(+) saccharose was conducted as described in examples 2 and 3.
In these cases, however, a film did not form but an amber coloured formless and fragile mass.

Example 6

*Preparation of a gel in three-dimensional matrix form*

The purification of chitosan and subsequent preparation of the chitosan solution were carried out as described in examples 1-4. The solution, contained in a mould in a 4 mm layer, was treated by freeze gelation, freezing at a temperature of -80°C in order to obtain a matrix with pores of 50 $\mu$m in diameter. When frozen in the suitable mould, the chitosan solution was immersed in an alcoholic solution of KOH (40% v/v of an aqueous solution of KOH in 96% v/v ethanol) previously cooled by placing it in a freezer until a temperature of -20°C was reached. The entire mixture was left in the freezer at -20°C for 24 hours. There followed a step to rehydrate the matrices obtained in this manner, by progressively replacing 250 microlitres/ml of potash solution with distilled water. The system was agitated until complete removal of the alcoholic potassium solution with distilled water.

Example 7

*Evaluation of viability and growth of endothelial cells on films obtained from chitosan alone; chitosan and salts; chitosan, trehalose and salts; chitosan, D-(+) raffinose and salts or chitosan, D-(+) saccharose and salts*

*a) Culture of endothelial cells*

HUVEC cells (cell lines derived from human umbilical cord endothelial cells) were cultivated in Medium 199, with antibiotics (penicillin 100 IU/ml and streptomycin 100 $\mu$g/ml) and 10% v/v fetal calf serum (FCS). Endothelial cell growth factor (ECGF) at a concentration of 50 $\mu$g/ml was also added to the medium. The cultures were thermostatically maintained at 37°C in water vapour saturated air and enriched with 5% $CO_2$. The cells were grown on plastic Petri dishes, and were inoculated onto the dish at a density of $10^4$ cells per $cm^2$. Any contamination by mycoplasmas were detected with a suitable kit (Boheringer, Mannheim, Germany).

*b) Trypsination and cell count*

After removing the culture medium, the cell monolayer was washed with a saline solution in phosphate buffer (PBS) at 37°C, to remove serum residues which could inhibit trypsin action. A solution of PBS containing 0.2% w/v of trypsin and 0.02% w/v of EDTA was then added. Cell detachment was monitored under a microscope, and after a few minutes of incubation at 37°C, the cells which detached from the substrate were collected from the plate and transferred into a Falcon 15 ml test tube containing medium with added 10% v/v FCS. The cells suspended in the test tube were centrifuged for 5 minutes at 1000xg; the supernatant was removed and the cells re-suspended in an adequate amount of complete medium. Finally the cells were counted under an optical microscope by means of a Burker hemocytometer, excluding dead cells by the use of Trypan Blue stain. Said stain only accumulates in cells with an altered membrane permeability.

*c) Seeding of cells onto chitosan film*

The cells were seeded onto 3 $cm^2$ films prepared as described in examples 1-4 at a density of $1 \times 10^4$ cells per $cm^2$. To allow an adequate cell adhesion, the films were deposited onto Petri dishes of suitable size and placed in an incubator for 1 hour. At the end of the incubation a quantity of medium sufficient to suitably cover the films was added to the dishes. At pre-established times the dishes were removed from the incubator and the cells on the films were analysed using a phase contrast microscope and photographed. One week after inoculation onto the chitosan films, these matrices were trypsinated, and the viable cells were counted under an optical microscope by means of a Burker hemocytometer, excluding dead cells by use of the Trypan Blue stain.

Figure 1 shows the cell growth curves (number of cells on 3 $cm^2$ as a function of time) on films of chitosan alone, chitosan containing salts, chitosan containing D-(+) raffinose and salts, or chitosan containing D-(+) saccharose and salts, compared with the cell growth on plastic dishes commonly used for cell cultures (Petri dishes, Corning). It was observed that cell growth on films of chitosan alone was comparable with the control up to 72 hours after seeding, thereafter a progressive reduction in cell numbers was noted. Regarding the other supports, no cell growth on the films of chitosan with salts and chitosan with trehalose and salts was apparent. Cell proliferation observed on films of chitosan with salts and D-(+) saccharose or D-(+) raffinose, respectively, was fully comparable with that obtained on Petri dishes.

Example 8

*Evaluation of the viability and growth of human fibroblasts on films obtained from chitosan alone; chitosan and salts; chitosan, trehalose and salts; chitosan, D-(+) raffinose and salts or chitosan, D-(+) saccharose and salts*

*a) Culture of fibroblasts*

WI-38 cells (human fetal fibroblasts) were cultivated in D-MEM (Dulbecco's modified Minimal Essential Medium) with added antibiotics (penicillin 100 IU/ml and streptomycin 100 $\mu$g/ml) and 10% v/v of fetal calf serum (FCS). The cultures were thermostatically maintained at 37°C in water vapour saturated air and enriched with 5% $CO_2$. The cells were grown on plastic Petri dishes, and were inoculated onto the dish at a density of $10^4$ cells per $cm^2$. Any contamination by mycoplasmas were detected with a suitable kit (Boheringer, Mannheim, Germany).

*b) Trypsination and cell count*

After removing the culture medium, the cell monolayer was washed with a saline solution in phosphate buffer (PBS) at 37°C, to remove serum residues which could inhibit the action of trypsin. A solution of PBS containing 0.2% w/v of trypsin and 0.02% w/v of EDTA was then added. Cell detachment was monitored under a microscope, and after a few minutes of incubation at 37°C, the cells which detached from the substrate were collected from the plate and transferred into a Falcon 15 ml test tube containing medium with added 10% v/v FCS. The cells suspended in the test tube were centrifuged for 5 minutes at 1000xg; the supernatant was removed and the cells re-suspended in an adequate amount of complete medium. Finally the cells were counted under an optical microscope by means of a Burker hemocytometer, excluding dead cells by the use of Trypan Blue stain. Said stain only accumulates in cells with an altered membrane permeability.

*c) Seeding of cells onto chitosan films*

The cells were seeded onto 3 cm$^2$ films prepared as described in examples 1-4 at a density of $1 \times 10^4$ cells per cm$^2$. To allow an adequate cell adhesion, the films were deposited onto Petri dishes of suitable dimensions and placed in an incubator for 1 hour. At the end of the incubation a quantity of medium sufficient to suitably cover the films was added to the dishes. At pre-established times the dishes were removed from the incubator and the cells on the films were analysed under a phase contrast microscope and photographed. One week after inoculation onto chitosan film, these matrices were trypsinated, and the viable cells were counted under an optical microscope by means of a Burker hemocytometer, excluding dead cells by use of Trypan Blue stain.

Figure 2 shows the cell growth curves (number of cells per cm$^2$ as a function of time) on films of chitosan alone, chitosan containing salts, chitosan containing D-(+) raffinose and salts, or chitosan containing D-(+) saccharose and salts, compared with cell growth on plastic dishes commonly used for cell culture (Petri dishes, Corning). It was observed that cell growth on films of chitosan alone was comparable with the control up to 72 hours after seeding, thereafter a progressive reduction in cell numbers was noted. Regarding the other supports, no cell growth on the films of chitosan with salts and chitosan with salts and trehalose was apparent. Cell proliferation observed on films of chitosan with salts and D-(+) saccharose or D-(+) raffinose, respectively, was fully comparable with that obtained on Petri dishes.

Example 9

*Characterization of gels in film form obtained from chitosan alone; chitosan and salts; chitosan, D-(+) raffinose and salts or chitosan, D-(+) saccharose and salts*

The gels in film form were viewed under a phase contrast optical microscope at a magnification of 100x. The images of films containing chitosan, chitosan and salts, chitosan, D-(+) raffinose and salts or chitosan, D-(+) saccharose and salts, prepared as described in examples 1-4 and recorded with a camera are shown in Figure 3. It was observed that the film of chitosan alone presented a supramolecular structure with microtrabeculae. In the film of chitosan and salts, the trabeculae were particularly accentuated. The films of chitosan, D-(+) saccharose or D-(+) raffinose and salts instead showed a substantially uniform surface indicating a lack of supramolecular organization. - Films prepared as in examples 3 and 4 were analysed by atomic force microscopy (Nanoscope IIIA, Digital Instruments) to determine their surface roughness characteristics. Said surface roughness is due to the depth of the depressions on the surface of the material. The chitosan films containing D-(+) saccharose and salts prepared as in example 3 had mean surface roughness values of 28.5 nm (standard deviation 4.5) while the chitosan film containing only salts prepared as in example 4 had mean surface roughness values of 195 nm (standard deviation 25).

The chitosan film containing D-(+) saccharose and salts prepared as in example 3 but without the final autoclave sterilization step had a mean surface roughness of 20.1 nm (standard deviation 6.5). This indicates that autoclave sterilization had no effect on this parameter. - The swelling index, $I_r$ in water was calculated in accordance with the following formula:

$$I_r = \frac{(p_r - p_s)}{p_s} \times 100$$

where $p_r$ is the weight of film swelled at equilibrium in water and $p_s$ is the dry film weight. The swelling index is an important parameter with regard to biomaterials in that it provides a measurement of the quantity of water that the material can retain in its interior, and, as a consequence, of its greater compatibility with the living material.

Table 1 shows the swelling indices obtained after having equilibrated in water the gels in film form prepared as given in examples 1-3 compared with a film prepared as given in example 2 or 3 in which D-(+) raffinose or D-(+) saccharose was substituted with D-(+) trehalose.

*Table 1: Swelling index (%) of gels in film form prepared according to examples 1-3 compared with a film prepared according to example 2 or 3 in which D-(+) raffinose or D-(+) saccharose was substituted with D-(+) trehalose*

| Gel composition | Chitosan | Chitosan, salts and D-(+) saccharose | Chitosan, salts and D-(+) raffinose | Chitosan, salts and D-(+) trehalose |
|---|---|---|---|---|
| Swelling index % | 342.7 | 1096.9 | 1285.3 | 772.9 |

The gels containing D-(+) raffinose or D-(+) saccharose showed swelling index values greater than 1000%.
[0071]   - Determination of the contact angle between water and the dried films of chitosan alone; chitosan, D-(+) saccharose and salts; chitosan, D-(+) raffinose and salts; chitosan, D-(+) trehalose and salts was conducted using a Lorentzen and Wettre instrument, recording the images with a camera. The data obtained are given in Table 2.

*Table 2: Contact angle (°) of dried films prepared according to examples 1-3 compared with a film prepared according to in example 2 or 3 in which D-(+) raffinose or D-(+) saccharose was substituted with D-(+) trehalose*

| Gel composition | Chitosan | Chitosan, salts and D-(+) saccharose | Chitosan, salts and D-(+) raffinose | Chitosan, salts and D-(+) trehalose |
|---|---|---|---|---|
| Contact angle ° | 59.5 | 38.7 | 27.4 | 28.4 |

The films prepared as in examples 2 and 3 with D-(+) raffinose and D-(+) saccharose presented, after drying, a contact angle value not greater than 22°.

## Example 10

*Evaluation of viability and growth of endothelial cells and fibroblasts in co-culture on the three-dimensional matrices obtained from chitosan, D-(+) raffinose and salts*

The culture, trypsination and cell count of fibroblasts and endothelial cells were conducted separately as described in examples 8 and 7, respectively.

*Seeding of cells onto the matrix*

The fibroblasts and endothelial cells were seeded simultaneously in a 1:1 ratio on the three-dimensional matrix prepared as described in example 6 at a density of $1 \times 10^4$ cells per $cm^2$. To allow an adequate cell adhesion, the matrix was deposited onto a Petri dish of 4.37 cm in diameter and placed in an incubator for 1 hour. At the end of the incubation a quantity of medium sufficient to suitably cover the matrix was added to the dish. Growth factors for the endothelial cells were added as described in example 7.
The plate was maintained in culture for 3 weeks, replacing the culture medium every 3 days. It was then removed from the incubator and the matrix with cells was fixed with 2.5% w/v glutaraldehyde in a 0.1 M sodium cacodylate buffer. After dehydration with absolute ethanol, the images of cells on the matrix were recorded using a scanning electron microscope (Figure 4). The formation of a continuous carpet of cells with the presence of tight junctions was observed (panels a-c). Furthermore, in panel d an area of matrix colonization by the cells, which emanate bridge-like extensions, was clearly visible.

## Claims

1. Process for preparing chitosan gels with stability and cytocompatibility comprising the following steps:

  a) preparing an acidified solution of chitosan;
  b) adding, in any order, the following components to the solution obtained in a): a sugar chosen from D-(+) saccharose and/or D-(+) raffinose, at least one phosphate salt, at least one alkaline or alkaline-earth metal chloride;

c) neutralizing the product obtained in b).

2.  Process as claimed in claim 1 wherein in step b), at least one monobasic phosphate salt and at least one dibasic phosphate salt are added.

3.  Process as claimed in claim 1 wherein the quantity of sugar added to solution a) is between 0.003 and 0.5 moles per litre of solution.

4.  Process as claimed in claim 1 wherein the quantity of sugar added to solution a) is between 0.03 and 0.4 moles per litre of solution.

5.  Process as claimed in claim 1 wherein the quantity of sugar added to solution a) is between 0.2 and 0.3 moles per litre of solution.

6.  Process as claimed in claim 1 wherein the sugar added is D-(+) saccharose in a quantity of 0.29 moles per litre of solution a), or D-(+) raffinose in a quantity of 0.20 moles per litre of solution a).

7.  Process as claimed in claims 1-6 wherein the chitosan concentration in solution a) is between 0.1 and 4% w/v.

8.  Process as claimed in claims 1-7 wherein the chitosan percentage in solution a) is between 1 and 3% w/v.

9.  Process as claimed in claims 1-8 wherein the chitosan percentage in solution a) is 2% w/v.

10. Process as claimed in claims 1-9 wherein solution a) is acidified with acetic acid or hydrochloric acid in a concentration of between 0.1 and 1.1 M.

11. Process as claimed in claims 1-10 wherein in step b) one or more of the following phosphates are added: $Na_2HPO_4$, $NaH_2PO_4$, $KH_2PO_4$.

12. Process as claimed in claims 1-11 wherein in step b) the following phosphate concentrations are added: $Na_2HPO_4$, from 0.1 to 35 mM; $NaH_2PO_4$, from 1 to 125 mM; $KH_2PO_4$, from 0.1 to 5 mM.

13. Process as claimed in claims 1-12 wherein in step b) one or more of the following chlorides are added: NaCl, KCl, $CaCl_2$ and $MgCl_2$.

14. Process as claimed in claims 1-13 wherein in step b) the following chlorides are added: NaCl, KCl, $CaCl_2$ and $MgCl_2$.

15. Process as claimed in claims 1-14 wherein in step b) the following chloride concentrations are added: NaCl, from 1 to 300 mM; KCl, from 0.1 to 5 mM; $CaCl_2$, from 0.1 to 2 mM; $MgCl_2$, from 0.1 to 1.5 mM.

16. Process as claimed in claims 1-15 wherein the gel is produced in the form of a film or three-dimensional matrix.

17. Composition useful for obtaining a stable and cytocompatible chitosan gel comprising: chitosan, a sugar chosen from D-(+) saccharose and/or D-(+) raffinose, at least one phosphate salt, and at least one alkaline or alkaline-earth metal chloride.

18. Stable and cytocompatible chitosan gel obtained by the process described in claims 1-16.

19. Gel as claimed in claim 18 in the form of a film or three-dimensional matrix.

20. Gel as claimed in claims 18-19 as a component of vascular prosthesis, bile duct prosthesis, material for filling bone defects, aids for wound repair or for the reconstruction of organs or tissues.

**Patentansprüche**

1. Verfahren zum Herstellen von Chitosangelen mit Stabilität und Cytokompatibilität, welches die nachfolgenden Schritte umfasst:

   a) Herstellen einer angesäuerten Lösung von Chitosan,
   b) Zugabe, in irgendeiner Reihenfolge, der nachfolgenden Bestandteile zu der in dem Schritt a) erhaltenen Lösung: ein Zucker ausgewählt aus D-(+)-Saccharose und/oder D-(+)-Raffinose, wenigstens ein Phosphatsalz, wenigstens ein Alkali- oder Erdalkalimetallchlorid,
   c) Neutralisieren des in dem Schritt b) erhaltenen Produkts.

2. Verfahren nach Anspruch 1, wobei in dem Schritt b) wenigstens ein monobasisches Phosphatsalz und wenigstens ein dibasisches Phosphatsalz zugegeben werden.

3. Verfahren nach Anspruch 1, wobei die Menge des zu der Lösung a) zugegebenen Zuckers zwischen 0,003 und 0,5 Mol pro Liter der Lösung beträgt.

4. Verfahren nach Anspruch 1, wobei die Menge des zu der Lösung a) zugegebenen Zuckers zwischen 0,03 und 0,4 Mol pro Liter der Lösung beträgt.

5. Verfahren nach Anspruch 1, wobei die Menge des zu der Lösung a) zugegebenen Zuckers zwischen 0,2 und 0,3 Mol pro Liter der Lösung beträgt.

6. Verfahren nach Anspruch 1, wobei der zugegebene Zucker D-(+)-Saccharose in einer Menge von 0,29 Mol pro Liter der Lösung a) oder D-(+)-Raffinose in einer Menge von 0,20 Mol pro Liter der Lösung a) ist.

7. Verfahren nach einem der Ansprüche 1 bis 6, wobei die Chitosanprozentsatz in der Lösung a) zwischen 0,1 und 4 % (Gew./Vol.) beträgt.

8. Verfahren nach einem der Ansprüche 1 bis 7, wobei der Chitosanprozentsatz in der Lösung a) zwischen 1 und 3 % (Gew./Vol.) beträgt.

9. Verfahren nach einem der Ansprüche 1 bis 8, wobei der Chitosanprozentsatz in der Lösung a) 2 % (Gew./Vol.) beträgt.

10. Verfahren nach einem der Ansprüche 1 bis 9, wobei die Lösung a) mit Essigsäure oder mit Chlorwasserstoffsäure in einer Konzentration zwischen 0,1 und 1,1 M angesäuert wird.

11. Verfahren nach einem der Ansprüche 1 bis 10, wobei in dem Schritt b) ein oder mehrere der nachfolgenden Phosphate zugegeben werden: $Na_2HPO_4$, $NaH_2PO_4$, $KH_2PO_4$.

12. Verfahren nach einem der Ansprüche 1 bis 11, wobei in dem Schritt b) die nachfolgenden Phosphatkonzentrationen zugegeben werden: zwischen 0,1 und 35 mM $Na_2HPO_4$, zwischen 1 und 125 mM $NaH_2PO_4$, zwischen 0,1 und 5 mM $KH_2PO_4$.

13. Verfahren nach einem der Ansprüche 1 bis 12, wobei in dem Schritt b) ein oder mehrere der nachfolgenden Chloride zugegeben werden: NaCl, KCl, $CaCl_2$ und $MgCl_2$.

14. Verfahren nach einem der Ansprüche 1 bis 13, wobei in dem Schritt b) die nachfolgenden Chloride zugegeben werden: NaCl, KCl, $CaCl_2$ und $MgCl_2$.

15. Verfahren nach einem der Ansprüche 1 bis 14, wobei in dem Schritt b) die nachfolgenden Chloridkonzentrationen zugegeben werden: zwischen 1 und 300 mM NaCl, zwischen 0,1 und 5 mM KCl, zwischen 0,1 und 2 mM $CaCl_2$, zwischen 0,1 und 1, 5 mM $MgCl_2$.

16. Verfahren nach einem der Ansprüche 1 bis 15, wobei das Gel in der Form eines Films oder einer dreidimensionalen Matrix hergestellt wird.

17. Zum Erhalten eines stabilen und cytokompatiblen Chitosangels geeignete Zusammensetzung enthaltend: Chitosan, einen Zucker ausgewählt aus D-(+)-Saccharose und/oder D-(+)-Raffinose, wenigstens ein Phosphatsalz und wenigstens ein Alkali- oder Erdalkalimetallchlorid.

18. Stabiles und cytokompatibles Chitosangel, welches durch ein in einem der Ansprüche 1 bis 16 beschriebenes Verfahren erhalten worden ist.

19. Gel nach Anspruch 18 in der Form eines Films oder einer dreidimensionalen Matrix.

20. Gel nach einem der Ansprüche 18 oder 19 als eine Komponente einer vaskulären Prothese, Gallengangprothese, Material zum Füllen von Knochendefekten, Hilfen zur Wundreparatur oder zur Rekonstruktion von Organen oder Geweben.

**Revendications**

1. Procédé de préparation de gels de chitosane stables et cytocompatibles comprenant les étapes suivantes :

   a) la préparation d'une solution acidifiée de chitosane ;
   b) l'addition, dans n'importe quel ordre, des composants suivants à la solution obtenue en a) : un sucre choisi parmi le D-(+)-saccharose et/ou le D-(+)-raffinose, au moins un sel de phosphate, au moins un chlorure de métal alcalin ou alcalinoterreux ;
   c) la neutralisation du produit obtenu en b).

2. Procédé selon la revendication 1, dans lequel dans l'étape b), au moins un sel de phosphate monobasique et au moins un sel de phosphate dibasique sont ajoutés.

3. Procédé selon la revendication 1, dans lequel la quantité de sucre ajoutée à la solution a) se situe entre 0,003 et 0,5 mole par litre de solution.

4. Procédé selon la revendication 1, dans lequel la quantité de sucre ajoutée à la solution a) se situe entre 0,03 et 0,4 mole par litre de solution.

5. Procédé selon la revendication 1, dans lequel la quantité de sucre ajoutée à la solution a) se situe entre 0,2 et 0,3 mole par litre de solution.

6. Procédé selon la revendication 1, dans lequel le sucre ajouté est du D-(+)-saccharose dans une quantité de 0,29 mole par litre de solution a), ou du D-(+)-raffinose dans une quantité de 0,20 mole par litre de solution a).

7. Procédé selon les revendications 1 à 6, dans lequel la concentration de chitosane dans la solution a) se situe entre 0,1 et 4 % p/v.

8. Procédé selon les revendications 1 à 7, dans lequel le pourcentage de chitosane dans la solution a) se situe entre 1 et 3 % p/v.

9. Procédé selon les revendications 1 à 8, dans lequel le pourcentage de chitosane dans la solution a) est de 2 % p/v.

10. Procédé selon les revendications 1 à 9, dans lequel la solution a) est acidifiée avec de l'acide acétique ou de l'acide chlorhydrique dans une concentration située entre 0,1 et 1,1 M.

11. Procédé selon les revendications 1 à 10, dans lequel dans l'étape b), un ou plusieurs des phosphates suivants sont ajoutés : $Na_2HPO_4$, $NaH_2PO_4$, $KH_2PO_4$.

12. Procédé selon les revendications 1 à 11, dans lequel dans l'étape b), les concentrations des phosphates suivantes sont ajoutées : $Na_2HPO_4$, de 0,1 à 35 mM ; $NaH_2PO_4$, de 1 à 125 mM ; $KH_2PO_4$, de 0,1 à 5 mM.

13. Procédé selon les revendications 1 à 12, dans lequel dans l'étape b), un ou plusieurs des chlorures suivants sont ajoutés : NaCl, KCl, $CaCl_2$ et $MgCl_2$.

**14.** Procédé selon les revendications 1 à 13, dans lequel dans l'étape b), les chlorures suivants sont ajoutés : NaCl, KCl, $CaCl_2$ et $MgCl_2$.

**15.** Procédé selon les revendications 1 à 14, dans lequel dans l'étape b), les concentrations des chlorures suivantes sont ajoutées : NaCl, de 1 à 300 mM ; KCl, de 0,1 à 5 mM ; $CaCl_2$, de 0,1 à 2 mM ; $MgCl_2$, de 0,1 à 1,5 mM.

**16.** Procédé selon les revendications 1 à 15, dans lequel le gel est produit sous la forme d'une matrice prenant la forme d'un film ou tridimensionnelle.

**17.** Composition utile pour l'obtention d'un gel de chitosane stable et cytocompatible comprenant : du chitosane, un sucre choisi parmi le D-(+)-saccharose et/ou le D-(+)-raffinose, où au moins un sel de phosphate et au moins un chlorure de métal alcalin ou alcalinoterreux.

**18.** Gel de chitosane stable et cytocompatible obtenu par le procédé décrit dans les revendications 1 à 16.

**19.** Gel selon la revendication 18, sous la forme d'une matrice prenant la forme d'un film ou tridimensionnelle.

**20.** Gel selon les revendications 18 et 19, en tant que composant d'une prothèse vasculaire, d'une prothèse de canal biliaire, de matériau pour combler des anomalies osseuses, des aides pour la réparation de plaies ou pour la reconstruction d'organes ou de tissus.

**Figure 1**

**Figure 2**

**Figure 3**

a

b

c

d

**Figure 4**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 62083875 B
- US 6719987 B
- WO 05089416 A
- JP 03151976 B
- CN 1628865
- EP 1246650 A
- JP 8269383 B
- JP 2004248949 B
- JP 11092504 B
- EP 1232203 A
- US 20030124172 A
- US 7022523 B
- WO 2005097871 A
- WO 2005079749 A
- US 6344488 B

**Non-patent literature cited in the description**

- **H.Shin et al.** *Biomaterials,* 2003, vol. 24, 4353-4364
- **Nerem.** *Ann. Biomed. Eng.,* 1991, vol. 19, 529-45
- *J Biomater Appl.,* 2005, vol. 20, 157-77
- *Biomaterials,* 2004, vol. 25, 3973-81
- **Rucker M et al.** *Biomaterials. 2006, Osteoarthritis Cartilage.,* 2005, vol. 13, 798-807
- *J Biomed Mater Res.,* 2002, vol. 59, 585-90
- *Biomaterials,* 2000, vol. 21, 2589-98
- *Biomaterials,* 2004, vol. 25, 779-85
- *Tissue Eng.,* 2005, vol. 11, 1105-14
- *J Biomed Mater Res A.,* 2006, vol. 77, 277-84
- **A. Di Martino et al.** *Biomaterials,* 2005, vol. 26, 5983-5990
- **Madihally et al.** *Biomaterials,* 1999, vol. 20, 1133-1142
- **Struszczyk.** Doctoral Thesis. University of Potsdam, November 2000
- **M.H. Ho et al.** *Biomaterials,* 2004, vol. 25, 129-138
- **S. V. Madihally ; H. W. T. Matthew.** *Biomaterials,* 1999, vol. 20, 1133-1142
- Pharmacopeia of the Italian Republic. 230